(19) **Europäisches Patentamt**

**European Patent Office**

**Office européen des brevets**

(11) **EP 0 759 781 B1**

(12) **EUROPEAN PATENT SPECIFICATION**

(45) Date of publication and mention
of the grant of the patent:
**25.01.2006 Bulletin 2006/04**

(51) Int Cl.:
*A61K 39/395* (2006.01)   *A61K 38/02* (2006.01)
*A61K 38/16* (2006.01)   *A61K 31/70* (2006.01)
*A61P 9/10* (2006.01)

(21) Application number: **95921563.3**

(22) Date of filing: **01.06.1995**

(86) International application number:
**PCT/US1995/006940**

(87) International publication number:
**WO 1995/033484 (14.12.1995 Gazette 1995/53)**

(54) **COMPOSITIONS FOR TREATING AND PREVENTING ATHEROSCLEROSIS**

ZUSAMMENSETZUNGEN ZUR BEHANDLUNG UND VORBEUGUNG VON ATHEROSKLEROSE

COMPOSES DESTINES AU TRAITEMENT ET A LA PREVENTION DE L'ARTERIOSCLEROSE

(84) Designated Contracting States:
**AT BE CH DE DK ES FR GB GR IE IT LI LU MC NL PT SE**

(30) Priority: **03.06.1994 US 253663**
**24.01.1995 US 377798**

(43) Date of publication of application:
**05.03.1997 Bulletin 1997/10**

(73) Proprietor: **The CBR Institute for Biomedical Research, Inc.**
**Boston MA 02115 (US)**

(72) Inventors:
• **WAGNER, Denisa D.**
**Wellesley, MA 02181 (US)**
• **JOHNSON, Robert C.**
**Watertown, MA 02172 (US)**

(74) Representative: **Polz, Leo et al**
**Hoffmann Eitle**
**Postfach 81 04 20**
**81904 München (DE)**

(56) References cited:
EP-A- 0 719 787   WO-A-92/16612
WO-A-93/24526   WO-A-93/24527
WO-A-94/05269   WO-A-94/05314
WO-A-95/03059

• **JOURNAL OF CLINICAL INVESTIGATION, Volume 91, issued June 1993, A.S. WEYRICH et al., "In Vivo Neutralization of P-Selectin Protects Feline Heart and Endothelium in Myocardial Ischemia and Reperfusion Injury", pages 2620-2629.**
• **NATURE, Volume 359, issued 29 October 1992, T. PALABRICA et al., "Leukocyte Accumulation Promoting Fibrin Deposition is Mediated in Vivo by P-Selectin On Adherent Platelets", pages 848-851.**
• **PROC. NATL. ACAD. SCI. U.S.A., Volume 88, issued August 1991, O.R. ETINGIN et al., "Identification of a Monocyte Receptor On Herpesvirus-Infected Endothelial Cells", pages 7200-7203.**
• **NATURE, Volume 364, issued 08 July 1993, M.S. MULLIGAN et al., "Protective Effects of Oligosaccharides in P-Selectin-Dependent Lung Injury", pages 149-151.**
• **CELL, Volume 74, issued 13 August 1993, T.N. MAYADAS et al., "Leukocyte Rolling and Extravasation Are Severely Compromised in P Selectin-Deficient Mice", pages 541-554.**
• **SCIENCE, Volume 258, issued 06 November 1992, L.A. LASKY, "Selectins: Interpreters of Cell-Specific Carbohydrate Information During Inflammation", pages 964-969.**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 759 781 B1

**Description**

<u>Field of the Invention</u>

This invention relates to compositions for the treatment and prevention of atherosclerosis.

<u>Background of the Invention</u>

Atherosclerosis is a principal cause of heart attacks, strokes and gangrene of the extremities. It has been reported that approximately 50% of all deaths in the United States, Europe and Japan are due to atherosclerosis. Atherosclerotic lesions can result from an excessive inflammatory-fibroproliferative response to various forms of insult to the endothelium and smooth muscle cells of the artery wall.

It is believed that the earliest type of atherosclerotic lesion is formed by binding of monocytes and T lymphocytes (CD4+ and CD8+) to the surfaces of endothelial cells in the lumen of the artery wall. These migrating cells proceed to penetrate beneath the arterial surface. The monocytes become macrophages, accumulate lipid, and become foam cells. These cells, together with the T lymphocytes, form a lesion called the fatty streak. The fatty streak subsequently develops into a fibrofatty intermediate lesion which is composed predominantly of layers of smooth muscle cells together with lipid-filled macrophages and T cells. These lesions in turn develop into complex occlusive lesions called fibrous plaques. The fibrous plaques can increase in size by projecting into the arterial lumen, and may thereby impede the flow of blood. Sudden death from myocardial infarctions can result from ruptures in the fibrous cap of the plaque, causing hemorrhage into the plaque, thrombosis and occlusion of the artery.

Current treatments for atherosclerosis include bypass grafting, endarterectomy, and angioplasty. These methods are high-risk invasive surgical procedures. Moreover, the failure rate of such treatments can often be high due to restenosis, which is thought to result from further inflammation, smooth muscle accumulation and thrombosis.

<u>Summary of the Invention</u>

It is an object of the invention to provide compositions for use in a safe, effective, easy and inexpensive method for treating or preventing atherosclerosis.

It is yet another object of the invention to provide compositions for use in a method for treating or preventing atherosclerosis which does not involve an invasive procedure.

It is yet another object of the invention to provide compositions for use in a simple method for treating or preventing atherosclerosis such as administering a pill, administering an injection or inserting an implant.

According to the invention the use of an agent according to the claims for the preparation of a pharmaceutical for treating or preventing atherosclerosis in a mammal is provided. An agent is provided for inhibiting interaction between P-selectin and a ligand of P-selectin. The agent is intended for administration to a mammal in need of such treatment to cause this inhibition to occur.

In certain embodiments, the P-selectin is on a cell, preferably an endothelial cell or a platelet. The ligand preferably is a carbohydrate, e.g., sialyl-Lewis x, sialyl-Lewis a, sialyl-Lewis x-pentasaccharide, polylactosaminoglycan, a carbohydrate containing 2,6 sialic acid, Lewis x 3'-0-sulfate, or heparin oligosaccharides, or a glycoprotein, e.g., PSGL-1, 160 kD monospecific P-selectin ligand, or lysosomal membrane glycoproteins. The ligand can be on, e.g., monocytes, neutrophils, eosinophils, CD4+ T cells, CD8+ T cells or natural killer cells.

The agent for use in the invention can be, e.g., a soluble form of a portion of the ligand ; an inhibitory carbohydrate, e.g., sialyl-Lewis x or its analogs, sialyl-Lewis a or its analogs, heparin oligosacchardies an inhibitory glycoprotein, e.g., PSGL-1, 160 kD monospecific P-selectin ligand, lysosomal membrane glycoprotein or glycoprotein containing sialyl Lewis X.

In certain embodiments, the agent inhibits interaction between P-selectin and the ligand so as to at least partially prevent formation of, or to at least partially reverse a formed, atherosclerotic fatty streak, and/or an intermediate lesion, and/or a fibrous plaque, or so as to at least partially prevent growth of an atherosclerotic lesion after a surgical procedure for preventing restenosis.

Variations of this method of this invention include the pharmaceutical in a form for administration prior to formation of an atherosclerotic lesion, subsequent to formation of an atherosclerotic lesion, and in a form for administration to a human.

Another aspect of the invention is the use of a therapeutic agent for the preparation of a pharmaceutical in a dosage form and concentration suitable for treating or preventing atherosclerosis in a mammal in need of such treatment, the agent being effective to inhibit interaction between P-selectin and a ligand of P-selectin.

The above and other objects, features and advantages of the present invention will be better understood from the following specification.

Detailed Description

This invention provides the use of an agent for the preparation of a pharmaceutical for treating or preventing atherosclerosis in a mammal. An agent is provided which inhibits interaction between P-selectin and a ligand of P-selectin. The agent is administered to a mammal in need of such treatment to cause this inhibition to occur.

Atherosclerosis is a condition which is meant to include the presence of any one or more types of atherosclerotic lesions on the surface of an arterial wall. Such lesions include fatty streaks, fibrofatty intermediate lesions and fibrous plaques. Atherosclerosis develops in many mammals. By mammals is meant human as well as non-human mammals. Treating atherosclerosis is meant to include preventing, arresting, altering, and reversing formation of atherosclerotic lesions.

P-selectin is a cell surface adhesion receptor. A receptor is a transmembrane protein with three major domains. The extracellular domain has an active site on the exterior side of the membrane which recognizes and binds to a ligand. A short hydrophobic domain makes up the transmembrane portion, and an intracellular cytoplasmic domain transmits a signal to the cell that the ligand has bound to the receptor. The extracellular domain of P-selectin includes a $Ca^{++}$-dependent C-type lectin domain, an epidermal growth factor-like domain, and a series of consensus repeats related to those of complement-binding proteins.

P-selectin is expressed in various cells, including endothelial cells and platelets. P-selectin mediates adhesion of different types of cells to each other. For example, P-selectin typically mediates heterotypic interactions of platelets or endothelial cells with blood cells. Cells which bind to P-selectin include monocytes, neutrophils, eosinophils, $CD4^+$ T cells, $CD8^+$ T cells and natural killer cells.

The binding of P-selectin to another cell can result from recognition of a ligand for P-selectin on that cell. By ligand is meant a moiety which binds to P-selectin, the moiety being either alone or attached to another molecule. P-selectin ligands include carbohydrate groups, e.g., sialyl-Lewis X (Foxall et al., J. Cell Biol., 117(4): 895-902, 1992; Polley et al., Proc. Nat'1 Acad. Sci., USA, 88:6224-6228, 1991) sialyl-Lewis a (Berg et al., J. Biol. Chem., 266: 14869-14875, 1991), sialyl-Lewis x pentasaccharide (Mulligan et al., Nature 364: 149-151, 1993), polylactosaminoglycan, carbohydrate containing 2,6 sialic acid (Larsen et al., J. Biol. Chem. 267: 11104-11110, 1992), Lewis x 3'-0-sulfate (Yuen et al., Biochemistry 31: 9126-9133, 1992) and heparin oligosaccharides (Nelson et al., Blood 82: 3253-3258, 1993). P-selectin ligands are also meant to include glycoproteins which contain a carbohydrate structure. For example, a P-selectin carbohydrate ligand can be linked to a mucin-like molecule. (Sako et al., Cell 75(6): 1179-1186, 1993; Linter et al., J. Biol. Chem. 125: 471-481, 1994). By mucin is meant serine- and threonine-rich proteins that are heavily O-glycosylated and have an extended structure. Other glycoprotein ligands include PSGL-1, 160 kD monospecific P-selectin ligand (Linter et al., J. Biol. Chem. 125: 471-481, 1994) and lysosomal membrane glycoproteins (Fukuda, J. Biol. Chem. 266: 21327-21332, 1991). Analogs of the above ligands which can bind to P-selectin, e.g., where fucose is replaced, e.g., by a diol group, or derivatives of the sialyl-Lewis x compounds which carry a $SO_3^-$ group instead of sialic acid, or contain a sialic acid in a 2,6 linkage, are also meant to be included as P-selectin ligands.

It is known that P-selectin is involved in cellular responses to inflammation resulting from injury or infection. This invention demonstrates that P-selectin can also be involved in the formation of atherosclerotic lesions. Example 1 shows that the presence in mice of a homozygous null mutation in P-selectin significantly decreases the size of the atherosclerotic lesions that are formed when the mice are fed a high fat diet, as compared to wild-type mice fed a high fat diet. The general health of these homozygous P-selectin deficient mice appear normal up to at least two years of age. The fact that these P-selectin deficient mice are viable, fertile, of normal size and vigor, and free of obvious signs of infection or disease, demonstrates that P-selectin is not required for normal development. The mouse fed a high fat diet, or various genetically engineered mice, are generally accepted as a good model for atherosclerosis in humans. (Lusis, Trends in Cardiovascular Medicine, 3: 135-143, 1993; Stoltzfus and Rubin, Trends in Cardiovascular Medicine, 3: 130-134, 1993; Ishida and Paigen, In Genetic Factors in Atherosclerosis; Monogr. Hum. Gen., Vol. 12: 189-222, 1989). Significantly, Example 4 shows that the presence in mice of a homozygous null P-selectin mutation also causes a significant reduction in the size of atherosclerotic lesions that are formed when the mice have in addition an LDL receptor-deficient mutation. Mice which lack LDL are a model system for the human disease called homozygous familial hypercholesterolemia (see Ishibashi et al., J. Clin. Invest., 93:1885-1893 (1994)), in which functional LDL receptor is absent, and as a consequence cholesterol-rich lipoproteins accumulate in the plasma, resulting in atherosclerotic lesions in childhood.

The agent for use in this invention can inhibit interaction between P-selectin and a ligand of P-selectin. By inhibiting interaction is meant, e.g., that P-selectin and its ligand are unable to properly bind to each other to effect proper formation of atherosclerotic lesions. Such inhibition can be the result of any one of a variety of events, including, e.g., preventing or reducing interaction between P-selectin and the ligand, inactivating P-selectin and/or the ligand, e.g., by cleavage or other modification, altering the affinity of P-selectin and the ligand for each other, diluting out P-selectin and/or the ligand, preventing surface, plasma membrane, expression of P-selectin or reducing synthesis of P-selectin and/or the ligand, synthesizing an abnormal P-selectin and/or ligand, synthesizing an alternatively spliced P-selectin and/or ligand, preventing or reducing proper conformational folding of P-selectin and/or the ligand, modulating the binding properties of P-selectin and/or the ligand, interfering with signals that are required to activate or deactivate P-selectin and/or the ligand, activating or deactivating P-selectin and/or the ligand at the wrong time, or interfering with other receotors, ligands or other molecules which are required for the normal synthesis or functioning of P-selectin and/or its ligand.

Examples of agents include soluble forms of the ligand, inhibitory carbohydrates, inhibitory glycoproteins, inhibitory glycopeptides, synthetic analogs of or the ligand.

The soluble form of the ligand, or a portion thereof, can compete with its cognate molecule for the binding site on the complementary molecule, and thereby reduce or eliminate binding between the membrane-bound P-selectin and the cellular ligand. The soluble form can be obtained, e.g., from purification or secretion of naturally occurring ligand, from recombinant ligand, or from synthesized ligand. Soluble forms of ligand are also meant to include, e.g., truncated soluble secreted forms, proteolytic fragments, other fragments, and chimeric constructs between at least a portion of ligand and other molecules. Soluble terms of P-selectin are described in Mulligan et al., J. Immunol., 151: 6410-6417, 1993, and soluble forms of P-selectin ligand are described in Sako et al., Cell 75(6): 1179-1186, 1993.

Inhibitory carbohydrates include oligosaccharides containing sialyl-Lewis a or sialyl-Lewis x or related structures or analogs, heparin oligosaccharides, e.g., heparin tetrasaccharides or low weight heparin, and other sulfated polysaccharides. Inhibitory carbohydrates are described in Nelson et al., Blood 82: 3253-3258, 1993; Mulligan et al., Nature 364: 149-151, 1993; Ball et al., J. Am. Chem. Soc. 114: 5449-5451, 1992; De Frees et al., J. Am. Chem. Soc. 115: 7549-7550, 1993. Inhibitory carbohydrates that are commercially available include, e.g., 3'-sialyl-Lewis x, 3'-sialyl-Lewis a, lacto-N-fucopentose III and 3'-sialyl-3-fucosyllactose, from Oxford GlycoSystems, Rosedale, NY.

Inhibitory glycoproteins, e.g., PSGL-1, 160 kD monospecific P-selectin ligand, lysosomal membrane glycoproteins, glycoprotein containing sialyl-Lewis x, (Suzuki et al., Biochem. Biophys. Res. Commun. 190: 426-434, 1993; Todderud et al., J. Leuk. Biol. 52: 85-88, 1992) that inhibit P-selectin interaction with its ligand can also be used in this invention.

Synthetic analogs or mimetics of or the ligand also can serve as agents.

Ligand analogs or mimetics include substances which resemble in shape and/or charge distribution the carbohydrate ligand for P-selectin. An analog of at least a portion of the ligand can compete with its cognate cellular ligand for the binding site on the P-selectin, and thereby reduce or eliminate binding between P-selectin and the cellular ligand. In certain embodiments which use a ligand analog, the sialic acid of a carbohydrate ligand is replaced with a group that increases the stability of the compound yet still retains or increases its affinity for P-selectin, e.g. a carboxyl group with an appropriate spacer. An advantage of increasing the stability is that it allows the agent to be administered orally. Sialyl-Lewis x analog with glucal in the reducing end and a bivalent sialyl-Lewis x anchored on a galactose residue via β-1,3- and β-1,6- linkages also inhibit P-selectin binding (DeFrees et al., J. Am. Chem. Soc., 115: 7549-7550, 1993).

Administration of the agent can be accomplished by any method which allows the agent to reach the target cells. These methods include, e.g., injection, deposition, implantation, suppositories, oral ingestion, inhalation, topical administration, or any other method of administration where access to the target cells by the agent is obtained. Injections can be, e.g., intravenous, intradermal, subcutaneous, intramuscular or intraperitoneal. Implantation includes inserting implantable drug delivery systems, e.g., microspheres, hydrogels, polymeric reservoirs, cholesterol matrices, polymeric systems, e.g., matrix erosion and/or diffusion systems and non-polymeric systems. e.g., compressed, fused or partially fused pellets. Suppositories include glycerin suppositories. Oral ingestion doses can be enterically coated. Inhalation includes administering the agent with an aerosol in an inhalator, either alone or attached to a carrier that can be absorbed.

Administration of the agent can be alone or in combination with other therapeutic agents. In certain embodiments, the agent can be combined with a suitable carrier, incorporated into a liposome, or incorporated into a polymer release system.

Preferably, protein agents are administered by intravenous or intramuscular injection; peptide agents by intravenous or intramuscular injection or by glycerin suppository; carbohydrate or sulfatide agents by intravenous or intramuscular injection, or with an aerosol in an inhalator; and synthetic analog agents by intravenous or intramuscular injection, or with an aerosol in an inhalator, or orally.

In certain embodiments of the invention, the administration can be designed so as to result in sequential exposures to the agent over some time period, e.g., hours, days, weeks, months or years. This can be accomplished by repeated administrations of the agent by one of the methods described above, or alternatively, by a controlled release delivery system in which the agent is delivered to the mammal over a prolonged period without repeated administrations. By a controlled release delivery system is meant that total release of the agent does not occur immediately upon administration, but rather is delayed for some time period. Release can occur in bursts or it can occur gradually and continuously. Administration of such a system can be, e.g., by long acting oral dosage forms, bolus injections, transdermal patches and sub-cutaneous implants.

Examples of systems in which release occurs in bursts include, e.g., systems in which the agent is entrapped in liposomes which are encapsulated in a polymer matrix, the liposomes being sensitive to a specific stimuli, e.g., temperature, pH, light or a degrading enzyme, and systems in which the agent is encapsulated by an ionically-coated microcapsule with a microcapsule core-degrading enzyme. Examples of systems in which release of the agent is gradual and continuous include, e.g., erosional systems in which the agent is contained in a form within a matrix, and diffusional systems in which the agent permeates at a controlled rate, e.g., through a polymer. Such sustained release systems can be, e.g., in the form of pellets or capsules.

The agent can be suspended in a liquid, e.g., in dissolved form or colloidal form. The liquid can be a solvent, partial solvent or non-solvent. In many cases water or an organic liquid can be used.

The agent can be administered prior to or subsequent to fibrous plaque formation. In certain embodiments, the agent is administered to patients, e.g., after angioplasty, stenting procedure, atherectomy, or bypass surgery or other vessel-corrective techniques, to aid in preventing restenosis. The agent also can be administered, preferably on a daily basis, to patients with familial hypercholesteremia, an early debilitating disease, who develop atherosclerotic lesions at a young age, often resulting in arterial narrowing and death.

The agent is administered to the mammal in a therapeutically effective amount. By therapeutically effective amount is meant that amount which is capable of at least partially preventing or reversing plaque formation. A therapeutically effective amount can be determined on an individual basis and will be based, at least in part, on consideration of the species of mammal, the mammal's size, the agent used, the type of delivery system used, the time of administration relative to plaque formation, and whether a single, multiple, or controlled release dose regimen is employed. A therapeutically effective amount can be determined by one of ordinary skill in the art employing such factors and using no more than routine experimentation.

Preferably, the concentration of an inhibitory glycoprotein or glycopeptide if applied systemically, is at a dose of about 0.1 to about 500 mg/kg body weight. Most preferably the dose is about 0.1 to about 5 mg/kg. The specific concentration partially depends upon the particular inhibitory, glycoprotein, or glycopeptide used, as some are more effective than others, Preferably, the concentration of a carbohydrate or a synthetic analog, if applied systemically is at a dose of about 0.01 to about 200 mg/kg body weight. Most preferably, the dose is about 0.1 to about 5 mg/kg.

The dosage concentration of the agent that is actually administered is dependent at least in part upon the final concentration that is desired at the site of action, the method of administration, the efficacy of the particular agent, the longevity of the particular agent, and the timing of administration relative to the formation of the atherosclerotic lesion. Preferably, the dosage form is such that it does not substantially deleteriously affect the mammal. The dosage can be determined by one of ordinary skill in the art employing such factors and using no more than routine experimentation.

The invention also includes the use of a therapeutic agent for the preparation of a pharmaceutical in a dosage form and concentration suitable for treating or preventing atherosclerosis in a mammal in need of such treatment, the agent being effective to inhibit interaction between P-selectin and its ligand.

EXAMPLES

Example 1: P-Selectin-Deficient Mice Fed a High Fat Diet Have Significantly Smaller Atherosclerotic Lesions Than Wild-Type Mice

This example illustrates that P-selectin plays an important role in the formation of atherosclerotic lesions in blood vessels. Comparisons were made of atherosclerotic lesions in wild-type and P-selectin-deficient mice fed a high fat diet. The P-selectin deficient mice contain a homozygous null mutation in P-selectin and were generated by homologous recombination in embryonic stem cells as described in Mayadas et al., Cell 74: 541-554, 1993.

Age-matched female wild-type and P-selectin deficient mice were used. (C57BL and 129 mixed background; both of these strains are susceptible to aortic lesion formation upon $\geq$ 14 week exposure to a high fat diet.). The mice were anesthetized and bled from the retroorbital venous plexus at the initiation of the prescribed diets. They were divided into two groups, each consisting of wild-type and P-selectin deficient mice. The control low fat group was fed Purina mouse chow containing 4.5% (w/w) animal fat, 0.03% (w/w) cholesterol, no sodium cholate and no casein. The other group was fed a high fat diet containing 15% (w/w) fat (from butter), 1.15-1.25% (w/w) cholesterol, 0.5% (w/w) sodium cholate and 20% casein (Rubin et al., Nature 353: 265-267 (1991)). The mice were started on the diets at 12-16 weeks of age and maintained on the diets for 19-21 weeks, at which time blood was drawn and the mice were sacrificed.

The total cholesterol levels in the blood plasma increased by comparable amounts in both P-selectin-deficient and wild-type mice. The p value is 0.46, indicating that there was no statistical difference in cholesterol levels in response to the high fat diet in the two sets of mice. The measured cholesterol value increases were similar to those reported by Paigen et al., Atherosclerosis, 57: 65-75, 1985.

The hearts were processed according to Paigen et al., Atherosclerosis, 68: 231-240 (1987). The heart and attached aorta were placed in 0.9% saline for 1 hour to remove erythrocytes and allow muscle relaxation. The hearts were then fixed in 10% buffered formalin and embedded in gelatin. For quantitative evaluation, the hearts were embedded in O.C.T. (optimal cooling temperature) compound, frozen and sectioned on a cryostat. Sections were discarded until reaching the junction of the heart muscle and aorta where the valve cusps become visible and the aorta is rounded. Unstained sections were regularly examined to locate the area of interest. This area of the aorta was shown previously to consistently result in lesions in C57BL/6 mice following 14 weeks exposure to the high fat diet. (Paigen et al., Atherosclerosis, 68: 231-240, 1987). Once the area was localized, four consecutive 10 $\mu$m sections were collected for each slide. Sectioning continued for approximately 350 $\mu$m (9-10 slides/heart) towards the aortic arch and exiting the valve region. Sections were collected onto gelatin coated glass slides and odd numbered slides were stained with oil red-O and hematoxylin. Tissues were then counterstained with light green.

One section on each of the odd numbered slides was assessed. Where possible, the same section on each of the five slides was used for quantitation. Thus, five sections, each 80 $\mu$m apart, were examined. If a section on a slide was folded or damaged, then the section immediately following or preceding replaced the flawed section. The slides were coded and the examiner was unaware of the genotype of the animal from which the sections originated. The size of the lesion was quantified using an ocular micrometer (net grid with 100 squares; each square 25 x 25 $\mu$m using 40x objective). Lesions less than 0.1 square using the 40x objective (400x magnification) were not counted. Lesions for each section were totaled. As shown in Table 1, the average size of the lesions in the P-selectin deficient mice fed a high fat diet was 3.6 times smaller than for the wild-type mice fed a high fat diet. No aortic lesions were present in wild-type or P-selectin deficient mice (one each) fed the low fat control diet.

TABLE 1 SIZE ($\mu$m$^2$) OF ATHEROSCLEROTIC LESIONS IN WILD TYPE AND P-SELECTIN-DEFICIENT MICE (five values per mouse, each 80 microns apart)

| Wild Type | P-Selectin-Deficient |
|---|---|
| 562.50 | 406.25 |
| 1375.00 | 1000.00 |
| 2000.00 | 562.50 |
| 562.50 | 687.50 |
| 1062.50 | 937.50 |
| | |
| 4750.00 | 0.00 |
| 2937.50 | 312.50 |
| 13000.00 | 1250.00 |
| 1375.00 | 187.50 |
| 0.00 | 750.00 |
| | |
| 0.00 | 62.50 |
| 2250.00 | 125.00 |
| 2250.00 | 437.50 |
| 2937.50 | 1125.00 |
| 0.00 | 750.00 |
| | |
| 1250.00 | 218.75 |
| 375.00 | 0.00 |
| 437.50 | 187.50 |
| 625.00 | 0.00 |
| 250.00 | 0.00 |
| | |
| 437.50 | 0.00 |
| 250.00 | 187.50 |
| 187.50 | 93.75 |
| 125.00 | 187.50 |
| 125.00 | 218.75 |
| | |
| 0.00 | 500.00 |

Table continued

| Wild Type | P-Selectin-Deficient |
|---|---|
| 0.00 | 625.00 |
| 0.00 | 9625.00 |
| 0.00 | 2812.50 |
| 0.00 | 437.50 |
| 812.50 | 0.00 |
| 875.00 | 187.50 |
| 4875.00 | 125.00 |
| 3812.50 | 187.50 |
| 4437.50 | 562.50 |
| 0.00 | 0.00 |
| 0.00 | 0.00 |
| 62.50 | 0.00 |
| 187.50 | 0.00 |
| 1312.50 | 0.00 |
| 5875.00 | |
| 2625.00 | |
| 6000.00 | |
| 6812.50 | |
| 7875.00 | |
| 4750.00 | |
| 5937.50 | |
| 5687.50 | |
| 9125.00 | |
| 437.50 | |

Statistical comparison of the lesion formation in the wild-type and P-selectin-deficient mice was done using the student t-test. Each mouse provided five individual values for statistical evaluation. Other investigators have previously determined that lesions 80 $\mu$m equidistant apart are likely to represent separate events and can therefore be computed separately. (Paigen et al, Atherosclerosis, 68: 231-240, 1987).

As Table 2 demonstrates, analysis of the atherosclerotic lesion data shows that the obtained t-statistic could have occurred by chance two times out of a thousand, and therefore the difference in the size of the lesions in the wild-type and P-selectin-deficient mice are highly statistically different.

TABLE 2 t-TEST: TWO-SAMPLE ASSUMING EQUAL VARIANCES

| | Wild Type | P-Selectin-Deficient |
|---|---|---|
| Mean | 2,212.50 | 618.75 |
| Variance | 8,306,760.20 | 2,405,408.65 |
| Observations | 50.00 | 40.00 |
| Pooled Variance | 5,691,388.49 | |
| Hypothesized Mean Difference | 0 | |
| df | 88 | |
| t Stat | 3.149 | |
| P(T<=t) two-tail | 0.002 | |

Example 2: Treating Atherosclerosis in a Human with Sialyl Lewis x

This example illustrates the use of an agent for the preparation of a pharmaceutical for treating for treating atherosclerosis in a human with an agent which inhibits interaction between P-selectin and its ligand. The patient is given an intramuscular injection of sialyl-Lewis x, once a day for a period of six months. (Mulligan et al., Nature 364: 149-151, 1993). The dose concentration per day is 1 mg/kg body weight. This treatment interferes with further development of atherosclerotic lesions.

Example 3: Mice Lacking LDL Receptor, a Mouse Model for Human Homozygous Familial Hypercholesterolemia, Develop Significantly Smaller Atherosclerotic Lesions If They Are Also Deficient in P-Selectin

This example illustrates that the absence of P-selectin can significantly attenuate the severe phenotype of heart disease in mice lacking LDL receptor -- a situation genetically identical to a human disease called homozygous familial hypercholesterolemia (FH). In humans with FH, the absence of functional LDL receptor leads to the accumulation of cholesterol-rich lipoproteins in plasma. As a consequence, macrophages loaded with cholesteryl esters are deposited throughout the body and atherosclerotic lesions of the aortic root and coronary arteries develop in childhood. (See Goldstein and Brown, Familial Hypercholesterolemia. In The Metabolic Basis of Inherited Disease, eds. Scriver et al., McGraw Hill Inc., N.Y. 1215-1250 (1989)).

To examine whether the absence of P-selectin can influence the development of the extensive atherosclerotic lesions in FH, the P-selectin-deficieni mice described in Example 1 (Mayadas et al., Cell 74:541-554 (1993)) were bred with LDL receptor-deficient mice developed through gene targeting technology described in Ishibashi et al., J. Clin. Invest., 92:883-893 (1993). The phenotype of the LDL receptor-deficient mice is remarkably similar to the phenotype of human homozygous FH when the animals are fed an atherogenic diet rich in cholesterol, saturated fat, and cholic acid (Ishibashi et al., J. Clin. Invest., 93:1885-1893 (1994)). Through the above-described breeding, a colony of mice deficient for LDL receptor and either wild-type for P-selectin or homozygous-deficient for P-selectin have been obtained. Twelve mice which are LDL receptor-deficient and wild-type for P-selectin (P-selectin-positive), and 11 mice deficient for both LDL receptor and P-selectin (P-selectin-negative), were put on an atherogenic diet for 8 weeks. Their hearts were then processed as described in Example 1. Within two weeks of the onset of the diet, their plasma cholesterol reached levels above 1,000 mg/dl, as compared to 200 mg/dl prior to the diet administration. At the time of sacrifice, a large sample of blood was collected for individual cholesterol, triglyceride and lipoprotein profile analysis. No differences were detected between the P-selectin-negative and P-selectin-positive mice. After 8 weeks on the high cholesterol diet, the mice had practically no HDL, and most of the cholesterol was in the LDL-VLDL region, in agreement with results reported by others (Isibashi et al., J. Clin. Invest., 93:1885-1893 (1994)). Importantly, there was no difference in the total plasma cholesterol levels between the P-selectin-positive and negative mice -- both groups gave approximately 1000 mg/dl (levels comparable to those seen in human FH) (Table 3).

TABLE 3 CHOLESTEROL LEVELS IN LDLR-DEFICIENT MICE AFTER 8 WEEKS ON HIGH-FAT DIET (mg/dl)

| P-Selectin Wild Type | P-Selectin-Deficient |
|---|---|
| 842 | 1068 |
| 1066 | 1053 |
| 1088 | 1228 |
| 1021 | 1076 |
| 940 | 1176 |
| 1241 | 1025 |
| 1135 | 795 |
| 1046 | 1114 |
| 926 | 1036 |
| 1842 | 1024 |
| 1438 | 1283 |
| 1280 | |

Statistics:

| | P-Selectin Wild Type | P-Selectin Deficient |
|---|---|---|
| mean | 1155.42 | 1079.82 |
| standard deviation | 272.09 | 127.94 |

Table continued

Statistics:

| | P-Selectin Wild Type | P-Selectin Deficient |
|---|---|---|
| n | 12 | 11 |
| p value | 0.411 | |

The results shown in Table 3 confirmed that the diet had the desired effect on plasma cholesterol level and also that the mice were correctly genotyped as LDL receptor-deficient. As described in Example 1, five sections of the aorta in the cusps regions were assessed. The mean area of the lesion was determined and this single value for each animal (Table 4) was used for statistical analysis (Tables 5 and 6).

TABLE 4 MEAN ATHEROSCLEROTIC LESION SIZE ($mm^2$) IN LDL RECEPTOR-DEFICIENT MICE (one value per mouse which is the mean from 5 sections, 80 microns apart)

| P-Selectin Positive (total) | P-Selectin Negative (total) |
|---|---|
| 0.267 | 0.082 |
| 0.149 | 0.189 |
| 0.283 | 0.207 |
| *0.256 | 0.241 |
| 0.276 | 0.154 |
| 0.182 | *0.240 |
| 0.253 | *0.079 |
| *0.436 | *0.157 |
| *0.279 | *0.209 |
| *0.177 | 0.100 |
| 0.097 | *0.044 |
| *0.336 | |

* indiates males

TABLE 5 t-TEST FOR P-SELECTIN-POSITIVE MICE AND P-SELECTIN-NEGATIVE MICE: TWO-SAMPLE ASSUMING EQUAL VARIANCES

| | P-Selectin Positive (total) | P-Selectin Negative (total) |
|---|---|---|
| Mean | 0.249 | 0.155 |
| Variance | 0.008 | 0.005 |
| Observations | 12.000 | 11.000 |
| Pooled Variance | 0.006 | |
| Hypothesized Mean Difference | 0.000 | |
| df | 21.000 | |
| t Stat | 2.810 | |
| P(T<=t) two-tail | 0.010 | |

TABLE 6 t-TEST FOR P-SELECTIN-POSITIVE MALE MICE AND P-SELECTIN-NEGATIVE MALE MICE: TWO-SAMPLE ASSUMING EQUAL VARIANCES

| | P-Selectin Positive (males) | P-Selectin Negative (males) |
|---|---|---|
| Mean | 0.296 | 0.146 |
| Variance | 0.009 | 0.007 |
| Observations | 5.000 | 5.000 |
| Pooled Variance | 0.008 | |
| Hypothesized Mean Difference | 0.000 | |
| df | 8.000 | |

Table continued

| | P-Selectin Positive (males) | P-Selectin Negative (males) |
|---|---|---|
| t Stat | 2.632 | |
| P(T<=t) two-tail | 0.030 | |

As shown in Table 4, the mean size of the atherosclerotic lesions in the P-selectin-positive mice was very large. Despite the overwhelming size of the atherosclerotic lesions in this FH model, the absence of P-selectin caused a significant reduction in lesion size (Table 5). This result was especially notable in males, where the lesions in the P-selectin-positive mice were twice the size of those found in P-selectin-negative animals (Table 6).

**Claims**

1. The use of an agent for inhibiting interaction between P-selectin and a ligand of P-selectin for the preparation of a pharmaceutical for treating or preventing atherosclerosis in a mammal, wherein the agent is selected from the group consisting of:

   - a carbohydrate selected from the group consisting of sialyl-Lewis x and its analogs, sialyl-Lewis a and its analogs, sialyl-Lewis x-pentasaccharide, polylactosaminoglycan, Lewis x 3'-O-sulfate and heparin oligosaccharides, or
   - a glycoprotein selected from the group consisting of PSGL-1, fragments of PSGL-1, synthetic analogs and mimetics of PSGL-1, 160 kD monospecific P-selectin ligand, lysosomal membrane glycoproteins and glycoproteins containing sialyl-Lewis x.

2. The use of claim 1 wherein said agent inhibits interaction between said P-selectin and said ligand so as to at least partially prevent formation of an atherosclerotic fatty streak.

3. The use of claim 1 wherein said agent inhibits interaction between said P-selectin and said ligand so as to at least partially prevent formation of an atherosclerotic intermediate lesion.

4. The use of claim 1 wherein said agent inhibits interaction between said P-selectin and said ligand so as to at least partially prevent formation of an atherosclerotic fibrous plaque.

5. The use of claim 1 wherein said agent inhibits interaction between said P-selectin and said ligand so as to at least partially prevent growth of an atherosclerotic lesion after a surgical procedure for at least partially preventing restenosis.

6. The use of claim 1 wherein said agent inhibits interaction between said P-selectin and said ligand so as to at least partially reverse a formed atherosclerotic fatty streak.

7. The use of claim 1 wherein said agent inhibits interaction between said P-selectin and said ligand so as to at least partially reverse a formed atherosclerotic intermediate lesion.

8. The use of claim 1 wherein said agent inhibits interaction between said P-selectin and said ligand so as to at least partially reverse a formed atherosclerotic fibrous plaque.

9. The use of claim 1 wherein the pharmaceutical is in a form for administration prior to formation of an atherosclerotic lesion.

10. The use of claim 1 wherein the pharmaceutical is in a form for administration subsequent to formation of an atherosclerotic lesion.

**Patentansprüche**

1. Verwendung eines Mittels zur Inhibition der Wechselwirkung zwischen P-Selektin und einem Liganden von P-Selektin zur Herstellung eines Arzneimittels zur Behandlung oder Prävention von Atherosklerose in einem Säugetier, worin das Mittel ausgewählt ist aus der Gruppe bestehend aus:

- einem Kohlenhydrat, ausgewählt aus der Gruppe bestehend aus Sialyl-Lewis-x und seine Analoga, Sialyl-Lewis-a und seine Analoga, Sialyl-Lewis-x-Pentasaccharid, Polylactosaminoglycan, Lewis-x-3'-O-Sulfat und Heparinoligosaccharide oder

- einem Glycoprotein, ausgewählt aus der Gruppe bestehend aus PSGL-1, Fragmente von PSGL-1, synthetische Analoga und Mimetica von PSGL-1, dem 160-kDmonospezifischen P-Selektinliganden, Glycoproteinen der lysosomalen Membran und Sialyl-Lewis-x enthaltende Glycoproteine.

2. Verwendung gemäß Anspruch 1, worin das Mittel die Wechselwirkung, zwischen dem P-Selektin und dem Liganden derart inhibiert, dass es wenigstens teilweise die Bildung eines atherosklerotischen Fettstreifens verhindert.

3. Verwendung gemäß Anspruch 1, worin das Mittel die Wechselwirkung zwischen dem P-Selektin und dem Liganden derart inhibiert, dass es wenigstens teilweise die Bildung einer atherosklerotischen intermediären Läsion verhindert.

4. Verwendung gemäß Anspruch 1, worin das Mittel die Wechselwirkung zwischen dem P-Selektin und dem Liganden derart inhibiert, dass es wenigstens teilweise die Bildung eines atherosklerotischen fibrösen Plaques verhindert.

5. Verwendung gemäß Anspruch 1, worin das Mittel die Wechselwirkung zwischen dem P-Selektin und dem Liganden derart inhibiert, dass es wenigstens teilweise das Wachstum einer atherosklerotischen Läsion nach einem chirurgischen Verfahren zur wenigstens partiellen Prävention einer Restenose verhindert.

6. Verwendung gemäß Anspruch 1, worin das Mittel die Wechselwirkung zwischen dem P-Selektin und dem Liganden derart inhibiert, dass es wenigstens teilweise einen ausgebildeten atherosklerotischen Fettstreifen zurückbildet.

7. Verwendung gemäß Anspruch 1, worin das Mittel die Wechselwirkung zwischen dem P-Selektin und dem Liganden derart inhibiert, dass es wenigstens teilweise eine ausgebildete atherosklerotische intermediäre Läsion zurückbildet.

8. Verwendung gemäß Anspruch 1, worin das Mittel die Wechselwirkung zwischen dem P-Selektin und dem Liganden derart inhibiert, dass es wenigstens teilweise einen ausgebildeten atherosklerotischen fibrösen Plaque zurückbildet.

9. Verwendung gemäß Anspruch 1, worin das Arzneimittel in einer Form zur Verabreichung vor der Bildung einer atherosklerotischen Läsion vorliegt.

10. Verwendung gemäß Anspruch 1, worin das Arzneimittel in einer Form zu Verabreichung nach der Bildung einer atherosklerotischen Läsion vorliegt.

**Revendications**

1. Utilisation d'un agent pour inhiber une interaction entre une P-sélectine et un ligand de P-sélectine pour la préparation d'un produit pharmaceutique pour traiter ou prévenir une athérosclérose chez un mammifère, dans laquelle l'agent est choisi dans le groupe constitué par :

- un hydrate de carbone choisi dans le groupe constitué par un sialyl-Lewis x et ses analogues, un sialyl-Lewis a et ses analogues, un sialyl-Lewis x-pentasaccharide, un polylactosaminoglycane, un Lewis x 3'-O-sulfate et des oligosaccharides d'héparine, ou

- une glycoprotéine choisie dans le groupe constitué par un PSGL-1, des fragments de PSGL-1, des analogues et mimes synthétiques de PSGL-1, un ligand de P-sélectine monospécifique 160 kD, des glycoprotéines de membrane lysosomale et des glycoprotéines contenant du sialyl-Lewis x.

2. Utilisation de la revendication 1 dans laquelle ledit agent inhibe une interaction entre ladite P-sélectine et ledit ligand de manière à au moins partiellement prévenir la formation d'une plaque graisseuse athérosclérotique.

3. Utilisation de la revendication 1 dans laquelle ledit agent inhibe une interaction entre ladite P-sélectine et ledit ligand de manière à au moins partiellement prévenir la formation d'une lésion intermédiaire athérosclérotique.

4. Utilisation de la revendication 1 dans laquelle ledit agent inhibe une interaction entre ladite P-sélectine et ledit ligand de manière à au moins partiellement prévenir la formation d'une plaque fibreuse athérosclérotique.

5. Utilisation de la revendication 1 dans laquelle ledit agent inhibe une interaction entre ladite P-sélectine et ledit ligand de manière à au moins partiellement prévenir la croissance d'une lésion athérosclérotique après une procédure chrirugicale pour au moins partiellement prévenir une resténose.

6. Utilisation de la revendication 1 dans laquelle ledit agent inhibe une interaction entre ladite P-sélectine et ledit ligand de manière à au moins partiellement renverser une plaque graisseuse athérosclérotique formée.

7. Utilisation de la revendication 1 dans laquelle ledit agent inhibe une interaction entre ladite P-sélectine et ledit ligand de manière à au moins partiellement renverser une lésion intermédiaire athérosclérotique formée.

8. Utilisation de la revendication 1 dans laquelle ledit agent inhibe une interaction entre ladite P-sélectine et ledit ligand de manière à au moins partiellement renverser une plaque fibreuse athérosclérotique formée.

9. Utilisation de la revendication 1 dans laquelle le produit pharmaceutique est sous une forme pour une administration avant la formation d'une lésion athérosclérotique.

10. Utilisation de la revendication 1 dans laquelle le produit pharmaceutique est sous une forme pour une administration ultérieure à la formation d'une lésion athérosclérotique.